# EUROPEAN PATENT APPLICATION

(11) **EP 1 645 612 A2**
(43) Date of publication of application: **12.04.2006**
(21) Application number: 05255506.7
(22) Date of filing: 08.09.2005
(51) Int. Cl.: C09K 11/85, G01N 23/00, G21K 4/00

(54) **Radiographic imaging device and radiographic imaging method**

(30) Priority: 15.09.2004 JP 2004268071
(71) Applicant: Konica Minolta Medical & Graphic, Inc., Tokyo 163-0512 (JP)
(72) Inventor: Yamashita, Hirobumi c/o Konica Minolta M&G. Inc.,, Hino-shi, Tokyo 191-8511 (JP); Hagiwara, Kiyoshi c/o Konica Minolta M&G. Inc.,, Hino-shi, Tokyo 191-8511 (JP)
(74) Representative: Rees, Alexander Ellison

(57) **Abstract**

A radiographic imaging device comprising a short-focus radiation source which radiates radiant rays to an examined object, a member to hold the examined object, and a radiographic image detector which detects radiant rays passing through the examined object and reads radiographic image information therefrom in order to perform phase contrast photography on the object which is held by the holding member, wherein
said detector is equipped with a phosphor plate which contains photostimulable phosphor particles having polyhedron crystal structures and the photostimulable phosphor occupies 60 to 80% of the photostimulable phosphor layer on the phosphor plate.

## Description

### BACKGROUND OF THE INVENTION

This invention relates to a radiographic imaging device and a radiographic imaging method.

X-ray radiography which uses X-ray which is one of radiant rays has been widely used for medical diagnostic imaging, nondestructive examinations, and so on. Radiographic images are shaded images which are made using that the transmittances of X-rays are dependent upon atomic weights of substances in the examined object. In other words, the radiography takes steps of causing the X-ray source to radiate X-ray, causing an X-ray detector to detect a 2-dimensional distribution of X-rays which passed an examined object and forming an X-ray image due to the X-ray absorption contrast of the examined object.

Recently, phase-contrast imaging has been proposed for X-ray radiography. This is also called refraction contrast imaging. This imaging method uses monochromatic parallel X-rays from a radiation source such as SPring-8 and X-rays from a micro-focus X-ray source whose focus size is about 10 µm. The images obtained by the phase-contrast imaging can have higher contrast in object boundaries than images having normal absorption contrasts only and consequently can be high fineness X-ray images.

The edge enhancement of the phase contrast images is accomplished by increasing the distance between an examined object and a radiographic image detector which detects X-rays passing through the examined object, refracting the X-rays when passing through the object, reducing the X-ray density inside the boundary of the object, and increasing the X-ray density outside the boundary of the object together with X rays which come directly without passing through the object.

Meanwhile, when the distance increases between the examined object and the radiographic image detector, the radiographic image taken becomes greater than the actual size of the examined object. However, images of actual object sizes are preferable for doctors to medically examine and locate the seat of an affliction. Therefore, it is preferable to take an enlarged phase contrast image of an object (of magnification M), reduce the enlarge image by 1/M (or a reciprocal of the magnification), and record the resulting image (of the actual object size) on a recording medium by an image recording device.

Such a radiographic image forming system which forms phase contrast images of actual object sizes is disclosed, for example, by Japanese Non-Examine Patent Publication 2001-238871. This system uses a radiographic imaging device which can freely change the distance between an object stand and a radiographic image detector for radiophotography, calculates a magnification from the distance, reduces the obtained image by the reciprocal of this magnification, and outputs the reduced image to an image output device.

In the above radiographic image forming system, however, the distance between the radiation source and the detector is greater than that in the conventional radiographic system in which the radiation source is close to the detector. Therefore, less radiant rays reach the detector and the resulting images cannot satisfy all of graininess, sharpness and high-quality without defects.

### SUMMARY OF THE INVENTION

An object of this invention is to provide a radiographic imaging device and a radiographic imaging method which can form radiographic images satisfying all of graininess, sharpness and high-quality without defects.

The above object of this invention can be accomplished by the structures below.
1. A radiographic imaging device comprising a short-focus radiation source which radiates radiant rays to an examined object, a member to hold the examined object, and a radiographic image detector which detects radiant rays passing through the examined object and reads radiographic image information therefrom in order to perform phase contrast photography on the object which is held by the holding member, wherein
   said detector is equipped with a phosphor plate which contains photostimulable phosphor particles having polyhedron crystal structures and the photostimulable phosphor occupies 60 to 80% of the photostimulable phosphor layer on the phosphor plate.
2. The radiographic imaging device of 1, wherein said photostimulable phosphor particles contain at least two different mean-particle-sizes of photostimulable phosphor particles.
3. The radiographic imaging device of 1, wherein the greater one of said 2 or more mean-particle-sizes of photostimulable phosphor particles is 6.0 to 10.0 µm and the smaller one is 3.0 to 5.0 µm.
4. The radiographic imaging device of 2, wherein the mixture ratio (by weight) of greater and smaller mean-particle-sizes of photostimulable phosphor particles is 95 : 5 to 50 : 50.
5. The radiographic imaging device of 1, wherein said photostimulable phosphor is represented by empirical formula (1) below.
   Empirical formula (1)
   Ba1-x M2x F Bry I1-y: aM1, bLn, cO
   where
   M1: at least one alkali metal atom selected from a group of Li, Na, K, Rb and Cs
   M2: at least one alkali earth metal atom selected from a group of Be, Mg, Sr and Ca
   Ln: at least one rare earth element selected from a group of Ce, Pr, Sm, Eu, Gd, Tb, Tm, Dy, Ho, Nd, Er and Yb
   x, y, a, b and c: 0 ≤ x ≤ 0.3, 0 ≤ y ≤ 1, 0 ≤ a ≤ 0.05, 0 < b ≤ 0.2, 0 < c ≤ 0.1
6. A radiographic imaging method of shooting by said radiographic imaging device of 1 and reducing the recorded magnified image to the actual object size before outputting it.

In accordance with the radiographic imaging device and method of this invention, the obtained phase-contrast radiographic images have excellent graininess, sharpness and defect-less image quality.

### BRIEF DESCRIPTION OF THE DRAWINGS

FIG. 1 shows a simplified system configuration of a radiographic imaging device of this invention.
FIG. 2 shows an example of preferred compressing process of this invention.

### BEST MODE FOR CARRYING OUT THE INVENTION

Below will be explained this invention in detail.

This invention relates to a radiographic (X-ray) imaging device comprising a short-focus radiation source which radiates radiant rays (X-rays) to an examined object, a member to hold the examined object, and a radiographic (X-ray) image detector which detects radiant rays passing through the examined object and reads radiographic image information therefrom in order to perform phase contrast photography on the object which is held by the holding member, wherein said (X-ray) detector is equipped with a phosphor plate which contains photostimulable phosphor particles having polyhedron crystal structures and the photostimulable phosphor occupies 60 to 80% of the photostimulable phosphor layer on the phosphor plate. Only this radiographic imaging device can satisfy all of graininess, sharpness and defect-less image quality of phase-contrast images, which is the object of this invention.

To heighten the effects of this invention, said photostimulable phosphor particles in the phosphor plate (of the radiographic (X-ray) image detector) should contain at least two different mean-particle-sizes of photostimulable phosphor particles. Further, for that purpose, the greater one of said mean-particle-sizes of photostimulable phosphor particles should be 6.0 to 10.0 µm and the smaller one is 3.0 to 5.0 µm and the mixture ratio (by weight) of greater and smaller mean-particle-sizes of photostimulable phosphor particles is 95 : 5 to 50 : 50.

Furthermore, to heighten the effects of this invention, said photostimulable phosphor should preferably be represented by the above Empirical formula (1).

The invention of 5 relates a radiographic imaging method which uses the radiographic imaging device of 1 to perform a phase contrast photography on examined objects.

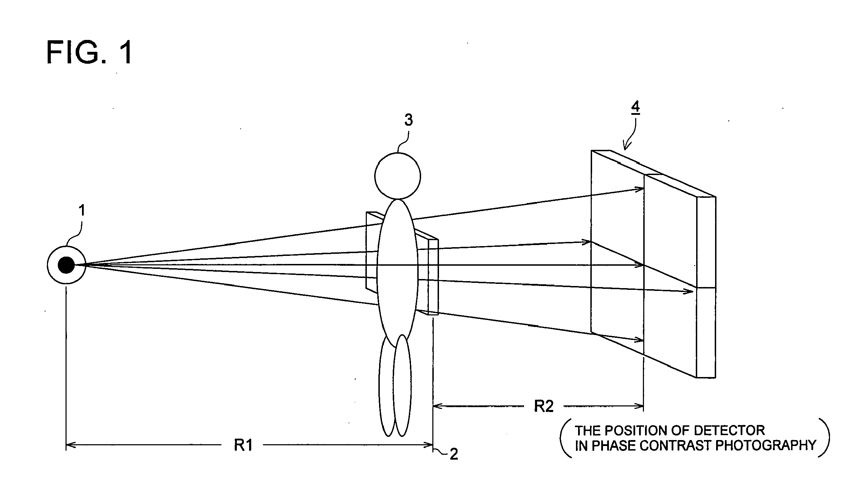

Referring to FIG. 1, the radiographic imaging device will be explained below.

Usually, the radiographic imaging device comprises X-ray source 1, object holding member 2 which determines the position of examined object 3 and secures the object, and X-ray detector 4. In this system, R1 indicates a distance between X-ray source 1 and secured object 3. R2 indicates a distance between object 3 and X-ray detector 4.

In this invention, X-ray detector 4 is equipped with a phosphor plate which contains photostimulable phosphor particles having a polyhedron crystal structure. The photostimulable phosphor occupies 60 to 80% (as the filling factor) of the photostimulable phosphor layer on the phosphor plate.

Meanwhile, the radiographic imaging method of this invention uses such a radiographic imaging device as describe above to perform phase contrast photography on objects to be examined.

The photostimulable phosphor particles having polyhedron crystal structures (herein called polyhedron particles) in this invention are three-dimensional particles each of which is a solid figure with four or more plane faces. They are for example, cube, octahedron, 14-hedron, tetrahedron, and so on. The polyhedrons of this invention are not limited to these.

The polyhedron particles can be prepared under the producing conditions below. In other words, this is a method of producing photostimulable of alkali earth metal fluoride halide with an activator of rare earth in a liquid phase into which oxygen is supplied. This method simultaneously performs a process of adding aqueous solution of inorganic fluoride into aqueous solution of barium halide to produce precursor crystals of photostimulable phosphor, as precipitate, which is rare-earth-activated alkali earth metal fluoride halide and a process of removing solvent from a solution containing barium of 3.3 mole/liter in the reactant mother liquid.

The filling factor of photostimulable phosphor in the photostimulable phosphor layer on the phosphor plate is calculated as explained below.

Phosphor is taken from the phosphor layer by peeling off the protective layer from the screen, dissolving the phosphor layer by methylethylketone, filtering, drying, and baking thereof at 600 °C for 1 hour to remove resin from the surface of phosphor. The filling factor of photostimulable phosphor is expressed by
Filling factor (V1%) = [O1 ÷ (P1 x Q1 x R1) ] x 100
where
- O1:: Weight of obtained phosphor (grams)
- P1:: Thickness of the phosphor layer (centimeters)
- Q1:: Screen area used (cm²),
- R1:: Specific gravity of phosphor (g/cm³)

The filling factor of this invention can be controlled for example by coating the surface of a base (backing) or undercoated base with photostimulable phosphor to form its layer, drying thereof under a desired condition, thus reparing a phosphor sheet having a photostimulable phosphor layer on it, and applying a pressure and heat to the phosphor sheet for example by passing the phosphor sheet between a highly-smooth nip roller of 1 to 100 cm in diameter and an opposite heating roller. This thermal compression of the phosphor sheet can increase the filling factor of photostimulable phosphor in the photostimulable phosphor layer.

A compressing method using calender rolls is not particularly limited. It can be for example a known compressing method which is described in "Resin Finishing Technology Handbook" (edited by Society of Polymer Science, Japan), published by THE NIKKAN KOGYO SHIMBUN, LTD. June 12, 1965. Further, the compressing method can use a pressing machine.

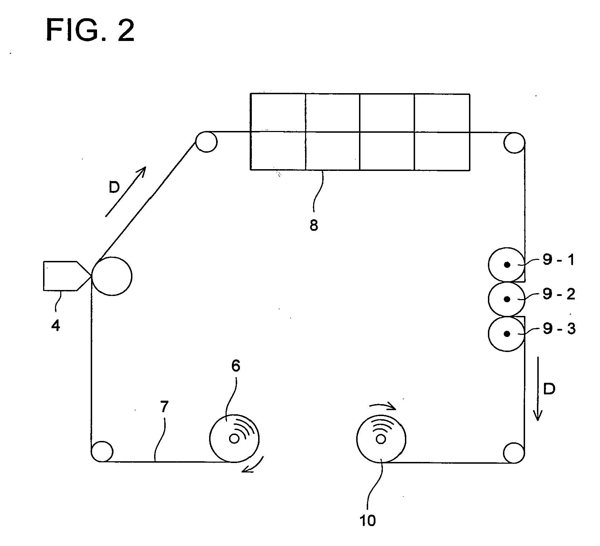

FIG. 2 shows an example of preferred compressing process of this invention.

In FIG. 2, this system feeds base sheet 7 (backing) from supply roller 6 in the direction of D, causes coater 4 to apply the photostimulable phosphor layer coating liquid to the surface of the base while running, guides the coated base into dry zone 8, dries the base by hot air from nozzles, compresses the dried base 7 (or the phosphor sheet) by a combination of calender rolls 9-1 to 9-3, and takes up the compressed base. In this system, it is preferable that calender rolls 9-1 and 9-3 are heating rolls and calender roll 9-2 is a plastic compliant roll.

The calender rolls are not limited in structure and resin type. However, a preferable calendar roller comprises an inner core of high-rigidity iron and an outer cylinder of hard-resin which covers the inner core. Typical calendar rolls are Elaglass (made by Kinyo Metal Co., Ltd.), Mirrortex rolls (Yamanouchi Rubber Co., Ltd.), and so on.

The compressing pressure of the calender rolls in this invention should preferably be 1.96 MPa to 49.0 MPa and more preferably 3.92 MPa to 24.5 MPa. These compressing conditions can improve the filling factor and smoothness of the photostimulable phosphor layer and consequently reduce irregularities on the surface of the photostimulable phosphor and sharpen the image. Particularly, the above compressing conditions can increase the compression rate of the phosphor layer near the base 7.

If the compressing pressure of the calender rolls is lower than 1.96 MPa and the temperature is lower than the glass transition point (Tg) of the polymer resin, the compression rate is insufficient and the smoothness of the phosphor is not good. If the compressing pressure is higher than 49.0 MPa and the temperature is higher than the softening point of the base, photostimulable phosphor particles may be broken and the base may be deformed. As the result, the luminance of the phosphor will not be recovered by re-baking. Therefore, these conditions are not preferable.

It is possible to form high-contrast radiographic images by using general medical X-ray tubes (used in medical care facilities) without using any huge synchrotron or micro-focus which generates weak X-rays.

In this case, a rotating anode tube is preferable as the X-ray tube. The rotating anode tube generates X-ray beams when electron beams emitted from the cathode collide with the anode.

The X-ray beams are incoherent like natural light. They are not parallel but divergent. The usual X-ray tube rotates the anode to make the service life of the anode longer since the anode may be damaged by heat if electron beams bombard a fixed area of the anode. When electron beams are made to collide with an anode surface of a preset size, X-rays generate from the anode surface and fly to an examined object. This anode surface of a preset size is called a "focus" when viewed along the movement of the X-rays. By using this focus size "D," the focus size of this invention can be measured by the half-power bandwidth in the intensity distribution of the radiation source. There are various focus shapes. The focus size D represents the length of one side of a square, the length of a shorter side of a rectangle or polygon, or the diameter of a circle.

The X-ray detector converts X-ray energy to the other energy so that it can be used as image information. Such X-ray detectors can be, for example, a screen (sensitizing paper) or film, a system using photostimulable phosphor (to be explained later), a system using a combination of X-ray phosphor and CCDs or CMOSs, and a system using a combination of X-ray phosphor or X-ray photoconductor and TFTs. This invention should preferably use an X-ray tube whose focus size is 30 µm or more.

Next will be explained photostimulable phosphor of this invention.

Preferable photostimulable phosphor of this invention is represented by Empirical formula (1) below.
Empirical formula (1)
Ba1-x M2x F Br y I1-y: aM1, bLn, cO
where ,
M1: at least one alkali metal atom selected from a group of Li, Na, K, Rb and Cs
M2: at least one alkali earth metal atom selected from a group of Be, Mg, Sr and Ca
Ln: at least one rare earth element selected from a group of Ce, Pr, Sm, Eu, Gd, Tb, Tm, Dy, Ho, Nd, Er and Yb
x, y, a, b and c: 0 ≤ x ≤ 0.3, 0 ≤ y ≤ 1, 0 ≤ a ≤ 0.05, 0 < b ≤ 0.2, 0 < c ≤ 0.1

A precursor producing method disclosed by Japanese Non-Examine Patent Publication H10-140148 and a precursor producing device disclosed by Japanese Non-Examine Patent Publication H10-147778 are available as preferable methods of producing photostimulable phosphor in this invention. The photostimulable phosphor precursor is a substance of Empirical formula (1) which has not been heat-treated at 600 °C or more and does not show any photostimulable and momentary illumination. This invention preferably use a liquid-phase synthetic method below to produce such a precursor

A solid-phase synthetic method is preferable to produce rare-earth activated alkali-earth-metal fluoride halide photostimulable phosphor of Empirical formula (1) in oxygen atmospherebecase a solid-phase synthetic method is hard to control particle shapes. Particularly, the liquid-phase synthetic method below is preferable to produce photostimulable phosphor.

A producing method comprising
a process of dissolving BaI₂, halide of rare-earth element (Ln), halide of alkali earthmetal (M2) when "x" is not 0 in Empirical formula (1), BaBr₂ when "y" is not 0, and halide of alkali metal (M1) to prepare a solution which contains BaI₂ of at least 3.3 moles/liter or preferably at least 3.5 moles/liter,
a process of keeping the above solution at 50 °C or higher or preferably 80 °C or higher, adding a solution of inorganic fluoride (ammonium fluoride or fluoride of alkali metal) of at least 5 moles/liter or preferably at least 8 moles/liter to the above solution, and precipitating rare-earth-activatied alkali-earth-metal fluoride iodide photostimulable phosphor precursor as crystals,
a process of removing solvent from the reaction liquid while adding inorganic fluoride to the solution,
a process of isolating the above precursor crystal precipitate from the reaction liquid, and
a process of calcinating the isolated precursor crystal precipitate without sintering.

This invention preferably uses monodispersive particles (crystals) having a mean particle size of 1 to 10 µm, preferably particles having a mean particle size of 1 to 5 µm and a distribution (%) of mean particle size of 20% or less, and more particularly, particles having a mean particle size of 1 to 3 µm and a distribution (%) of mean particle size of 15% or less.

The mean particle size in this invention is defined as an average obtained by dividing the total volumetric diameters of 200 particles (selected at random using electron microscopic pictures) by the number of particles (200).

Next will be explained a method of producing the photostimulable phosphor.

### (Preparation of precursor crystal precipitate and photostimulable phosphor)

First, raw material compounds excluding fluorine compounds are dissolved in a water-based medium. In other words, BaI₂, halide of rare-earth element (Ln), halide of alkali earthmetal (M2) if necessary, and halide of alkali metal (M1) were fully mixed in a water-based medium to dissolve. Here it is necessary to control the concentration of BaI₂ and the quantity of water-based medium in advance so that the concentration of BaI₂ may be at least 3.3 moles/liter or preferably 3.5 moles/liter or more.

In this case, if the barium concentration is low, a precursor of the expected composition cannot be obtained or the particles may be greater than expected. Therefore, the barium concentration must be selected adequately. After a careful study and research, we, inventors, found that the barium concentration of 3.3 moles/liter or more can form fine precursor particles.

It is possible to add a little acid, ammonia, alcohol, water-soluble high polymer, and fine power of water-insoluble metal oxide if necessary. Further, it is also preferable to add an adequate quantity of lower alcohol (methanol or ethanol) to the solution. The quantity of lower alcohol must not be much enough to reduce the solubility of BaI₂. This aqueous solution (or the reaction mother liquid) is kept at 80 °C.

Next, an aqueous solution of inorganic fluoride (such as ammonium fluoride and fluoride of alkali metal) is added to the above aqueous solution which is stirred continuously at 80 °C. It is preferable to add the inorganic fluoride solution to a solution part which is stirred vigorously. The rare-earth-activated alkali-earth-metal fluoride halide phosphor precursor of Empirical formula (1) is produced by this addition of the inorganic fluoride solution to the reaction mother liquid and precipitates as crystals.

In this invention, the solvent is removed from the reaction liquid while the aqueous solution of inorganic fluoride is added. The solvent can be removed from the reaction liquid any time while the aqueous solution of inorganic fluoride is added to the reaction liquid. It is preferable that the entire solution weight after solvent removal doe not exceed 0.97 of the entire solution weight before solvent removal (or the sum of the weight of the reaction mother liquid and the weight of the added aqueous solution). (This ratio is called a solvent removal ratio.) If this ratio exceeds 0.97, BaFI may not fully grow up into crystals. Therefore the ratio should preferably be 0.97 or less and more preferably 0.95 or less. If the solvent is removed too much, the reaction solution becomes too viscous to be handled easily.

Accordingly, the preferable solvent removal ratio is down to 0.5. Further, since a time period required for solvent removal greatly affects the productivity of the photostimulable phosphor and the solvent removing method will affect the shape and size distribution of resulting particles, an adequate solvent removing must be selected. A general method of removing a solvent from an aqueous solution is heating the solution to evaporate the solvent. This method is also available to this invention. The solvent removal facilitates preparation of a precursor of expected compositions. It is preferable to use the other solvent removing method to increase the productivity and to shape particles adequately. In this case, any solvent and method can be used. It is also possible to use a method of using a separation membrane such as a reverse osmosis membrane. Judging from the productivity, this invention preferably uses a solvent removing method below.
1. This method uses an enclosed type reaction vessel which has at least two ventilation holes to pass dry air. Any dry air can be used but air or nitrogen gas is preferable for safety's sake. The solvent is carried away by the dry air due to the saturated vapor concentration of the air. In addition to ventilate the void of the reaction vessel, it is also effective to feed air into the liquid phase in the reaction vessel to make air bubbles in the liquid and cause the bubbles to contain the solvent.
2. This method is done in a reduced-pressure status. As is well known, when the pressure goes down, the vapor pressure of the solvent also goes down. Therefore the pressure reduction can speed up solvent removal. The degree of pressure reduction can be selected freely according to the kind of solvent. For example, when the solvent is water, the preferable pressure reduction is 86 kPa or less.
3. This method increases the evaporation area of the liquid surface to remove the solvent efficiently. When heating and stirring are made to advance a liquid-phase reaction in a reaction vessel of a predetermined volume as in this invention, usually a heating means is immersed in the liquid or provided on the outer wall of the reaction vessel. In this method, the heat transfer is performed only in an area where the liquid is in contact with the heating means. The hear transfer area reduces as the solvent evaporation advances and consequently, it takes more time to remove the solvent. To prevent this, it is effective to increase the heat transfer area by scattering the solution onto the inner wall of the reaction vessel by a pump or stirrer. This method is known as a "wetted wall" method. In addition to the wetted-wall method using a pump, a wetted-wall method using a stirrer is disclosed by Japanese Non-Examine Patent Publications H06-335627 and H11-235522.

These methods can be used singly or in combination. For example, effective methods are a combination of a method of forming a liquid membrane and a method of reducing the pressure in the reaction vessel and a combination of a method of forming a liquid membrane and a method of feeding dry air into the reaction vessel. Particularly, the former method is preferable. Such a method is disclosed by Japanese Non-Examine Patent Publication H06-335627 and Patent Application Laid-Open Disclosure 2002-35202.

The method separates produced from the solution by filtration or centrifugal separation, washes the crystals with methanol or the like, and dries them up. Then, adds a sintering preventor such as fine alumina or silica powder to the dried precursor crystals, and fully mixes the mixture to uniformly coat the crystal surfaces with the sintering preventor. It is possible to omit the addition of the sintering preventor when the sintering conditions are satisfied.

Then, this method puts the phosphor precursor crystals in a heat-resistant vessel such as a quartz boat, alumina crucible, or quartz crucible, puts the heat-resistant vessel in the center of an electric furnace, and calcinates the phosphor precursor crystals without sintering. The calcinating temperature should preferably be 400 to 1,300 °C and more preferably 500 to 1,000 °C. The calcinating time is dependent upon the quantity of the phosphor precursor crystals in the boat or crucible, the calcinating temperature, and take-up temperature, but it is preferably 0.5 to 12 hours.

The gas atmosphere in the furnace for calcinations can be
a neutral gas atmosphere (such as nitrogen gas atmosphere and argon gas atmosphere),
a weak-reducing atmosphere (such as a nitrogen gas atmosphere containing a little hydrogen gas and a carbon dioxide gas atmospherecontaining carbon monoxide), or
an atmosphere containing a trace of oxygen. A preferable calcination method is disclosed by Japanese Non-Examine Patent Publication 2000-8034. The above calcinations finishes the target photostimulable phosphor of oxygen-added rare-earth-activated alkali earth metal fluoride halide. A radiographic image conversion panel is produced which contains a phosphor layer formed using this photostimulable phosphor.

The radiographic image conversion panel uses various kinds of polymer materials for its base (backing). Particularly the materials are preferable if they can be processed into flexible sheet or web that can be used as information recording material. Judging from this, preferable films are cellulose acetate, polyester, polyethylene terephthalate, polyethylene naphthalate, polyamide, polyimide, triacetate, and polycarbonate films.

The thickness of the base layer is dependent upon the material of the base but generally it is 80 µm to 1,000 µm and more preferably 80 µm to 500 µm for convenience in handling. The surface of the base can be smooth or matted to increase the adhesion of the base to the photostimulable phosphor layer.

The base (backing) can have an undercoat layer to increase the adhesion between the base and the photostimulable phosphor layer.

The undercoat layer of this invention should preferably contain a crosslinkable polymer resin and its crosslinking agent.

Any polymer resin can be used for the undercoat layer. It can be, for example, polyurethane, polyester, vinyl chrolide copolymer, vinyl chrolide-vinyl acetate copolymer, vinyl chrolide-vinylidene chloride copolymer, vinyl chrolide acrylonitrile copolymer, butadiene-acrylonitrile copolymer, polyamide resin, polyvinyl butyral, cellulose derivative (such as nitrocellulose), styrene-butadiene copolymer, a variety of synthetic rubber resins, phenol resin, epoxy resin, urea resin, melamine resin, phenoxy resin, silicone resin, acrylic resin, urea formamide resin, and so on. Among them, polyurethane, polyester, vinyl chrolide copolymer, polyvinyl butyral, and nitro-cellulose are preferable. Further, the mean glass transition temperature (Tg) of polymer resin for the undercoat should be 25 °C or higher and more preferably 25 to 200 °C.

The crosslinking agent for the undercoat layer in this invention can be any crosslinking agent. It can be, for example, multifunctional isocyanate and its derivative, melamine and its derivative, amino resin and its derivative, and so on. Among them, multifunctional isocyanate compounds are preferable as the crosslinking agent. Commercially available multifunctional isocyanate compounds are, for example, Collonate HX and Collonate 3041 (by Nippon Polyurethane).

This invention uses a method below to form the undercoat layer on the base (backing).

First, this method prepares an undercoated layer coating liquid by adding a polymer resin and a crosslinking agent which are selected from the above to an adequate solvent, for example, a solvent which is used for preparation of a phosphor layer coating liquid (to be explained later) and fully mixing thereof.

The quantity of the crosslinking agent to be used is dependent upon the characteristics of a target radiographic image conversion panel, raw materials for photostimulable phosphor layers and the bases, and polymer resins for undercoat layers. To assure the adhesion of the photostimulable phosphor layer to the base, the weight of the crosslinking agent should be up to 50% and preferably 15 to 50% of the weight of the polymer regin.

The thickness of the undercoat layer is dependent upon the characteristics of a target radiographic image conversion panel, raw materials for photostimulable phosphor layers and the bases, and polymer resins for undercoat layers. Generally, the thickness should preferably be 3 to 50 µm and more preferably 5 to 40 µm.

Typical binders for the phosphor layer in this invention are, for example, proteins (such as gelatine), polysaccharide (such as dextran), or natural polymer substances (such as gum arabic) and synthetic high polymer substances (such as polyvinyl butyral, polyvinyl acetate, nitrocellulose, ethylcellulose, vinylidenechloride-vinylchrolide copolymer, polyalkyl methacrylate, vinylchrolide-vinylacetate copolymer, polyurethane, cellulose acetate butylate, polyvinyl alcohol, and linear polyester). However, it is preferable that the binder is a resin which contains thermoplastic elastomer as a main ingredient. Such thermoplastic elastomers are, for example, polystyrene-related, polyolefin-related, polyurethane-related, polyester-related, polyamide-related, polybutadiene-related, ethylene vinyl acetate related, polyvinyl chrolide related, natural rubber related, fluorine rubber related, polyisoprene-related, chlorinated polyethylene related, styrene-butadiene rubber and silicone rubber related thermoplastic elastomers. Amon the above thermoplastic elastomers, polyurethane- and polyester-related thermoplastic elastomers are preferable because they have a strong bonding force with phosphor, a good dispersibility, and a good ductility. They are preferable because they can increase the bendng resistance of the sensitizing screen. Here, these binders can be cross-linked by proper crosslinking agents.

The mixing ratio of the binder and the photostimulable phosphor in the coating liquid is dependent upon the Haze index setting of the radiographic image conversion panel, but preferably the binder should be 1 to 20 mass parts and more preferably 2 to 10 mass parts of the photostimulable phosphor.

The radiographic image conversion panel having a coated phosphor layer can be coated with a film for protection. Such films can be polyester, polymethacrylate, nitrocellulose film, and cellulose acetate films, each of which has an excitation-light absorption layer whose Haze index is 5% to 60% (not including 60%) (measured by a method defined in ASTMD-1003). Drawn polyethylene terephthalate films and polyethylene naphthalate films are preferable as such protective films judging from film transparency and strength. Further, judging from moisture-proofing, the above films should preferably be coated with a metal oxide or silicon nitride film by vacuum evaporation.

The Haze index of a film to be used for the protective layer can be easily controlled by the Haze index of a resin film to be used. Industrial resin films of arbitrary Haze indexes can be easily obtained. Usually, the protective film for the radiographic image conversion panel must be optically very transparent. Various high-transparency plastic films (having a Haze index of 2 to 3%) are also available commercially.

To increase the advantageous effects of this invention, the haze index of the film should be 5% to 60% (not including 60%) and more preferably 10% to 50% (not including 50%). If the Haze index is less than 5%, image irregularities and linear noises can be eliminates less effectively. If the Haze index is 60% or more, the effect of image sharpness will be deteriorated.

The film used for the protective layer in this invention can have an optimum moisture-proof property by laminating a plurality of resin films or deposited films (on which metal oxide or the like is vapor-deposited according to the required moisture-proofing. To prevent deterioration of the photostimulable phosphor by moisture absorption, the water vapor permeability of the protective film should be at least 5.0 g/m² a day or less. The resin film can be laminated by any known laminating method.

It is preferable to provide an excited-light absorbing layer between the laminated resin films in order to protect the phosphor plate against physical impacts and chemical deterioration. With this, the phosphor plate can keep its performance steadily for a long time. A plurality of excited-light absorbing layers can be provided. Further, a colored adhesive layer to laminate resin films can be used instead of the excited-light absorbing layer.

The protective film can be bonded to the photostimulable phosphor layer by means of an adhesive layer. However, it is more preferable that the protective film is provided to cover (or seal) the phosphor surface. (This structure is also called a sealing structure.) The phosphor plate can be sealed by any known method. It is preferable to use a moisture-proof protective film whose outermost resin layer to be contact with the phosphor plate is made of a heat-sealingresin film. This facilitates sealing of the protective film to the phosphor plate and can increase the sealing efficiency. This is one of the preferred embodiments of this invention.

Further, it is preferable to sandwich the phosphor sheet between two moisture-proof protective films and heat-seal the protective film edges that run off the edges of the phosphor sheet by an impulse sealer or the like. This can prevent invasion of water into the phosphor sheet from its edges. Further it is preferable to use a laminated moisture-proof film containing one or more aluminum films instead of a moisture-proof protective film facing to the base surface. This aluminum-laminated film can completely prevent water invasion and facilitate sealing works. Furthermore, it is preferable to seal the protective films by the impulse sealer in a vacuum environment because this can prevent displacement of the phosphor sheet in the envelope of the protective films and exclude moisture from inside the envelope.

The outermost heat-sealing resin layer of the moisture-proof protective film which faces to the phosphor surface need not be bonded to the phosphor surface. "Not bonded to the phosphor surface" means that the phosphor surface is assumed to be optically and mechanically discontinuous to the moisture-proof protective film even when the phosphor surface is microscopically in point-contact with the protective film. Here, the above heat-sealing resin films indicate resin films that can be heat-sealed by a general-purpose impulse sealer. They can be, for example, ethylene vinyl acetate copolymer (EVA), polypropylene (PP), and polyethylene (PE) films.

Organic solvents that can be used for preparation of the phosphor layer coating liquid can be, for example,
lower alcohols (such as methanol, ethanol, isopropanol, and n-butanol),
ketones (such as acetone, methylethyl ketone, methyl isobutyl ketone, and cyclohexanon),
esters of lower alcohol and lower fatty acid (such as methyl acetate, ethyl acetate, and n-butyl acetate),
ethers (such as dioxane, ethylene glycol monoethyl ether, and ethylene glycol monomethyl ether),
aromatic compounds (such as triol and xylol),
halogenated hydrocarbons (such as methylene chloride and ethylene chloride), and
their mixtures.

The coating liquid can contain various additives such as a dispersing agent to improve the dispersibility of particles in the coating liquid and a plasticizer to increase the binding force between the binder and phosphor particles in the prepared photostimulable phosphor layer.

Such dispersing agents can be, for example, phthalic acid, stearic acid, caproic acid, and oleophilic surfactant. Such plasticizers can be, for example,
ester phosphates (such as triphenyl phosphate, tricresyl phosphate, and diphenyl phosphate),
ester phthalates (such as diethyl phthalate and dimethoxy ethyl phthalate),
ester glycolates (such as ethyl phthalyl ethyl glycolate and butyl phthalyl butyl glycolate), and
polyesters of polyethylene glycol and aliphatic dibasic acid (such as polyester of triethylene glycol and adipic acid and polyester of diethylene glycol and succinic acid).

Further, the photostimulable phosphor layer coating liquid can contain a dispersing agent (such as stearic acid, phthalic acid, caproic acid, and oleophilic surfactant) to improve the dispersibility of photostimulable phosphor particles.

A dispersing device such as a ball mill, bead mill, sand mill, atraiter, 3-roll mill, high-speed impeller disperser, Kady mill, ultrasonic disperser is used to prepare a coating liquid for the photostimulable phosphor layer.

The coating liquid prepared by the above disperser is evenly applied to the surface of the base (to be explained later).

A coating method can be a normal coating means such as Doctor Blade, roll coater, knife coater, comma coater, and lip coater.

The liquid layer coated on the base by the above means is heated and dried. This is the photostimulable phosphor layer formed on the base. The thickness of the photostimulable phosphor layer is dependent upon the property of the target radiographic image conversion panel, kind of the photostimulable phosphor, mixing ratio of binder and phosphor, and so on. However, the layer thickness is usually 10 to 1,000 µm and preferably 10 to 500 µm.

### [Embodiment]

In the following examples will be described several preferred embodiments to illustrate the invention. However, it is to be understood that the invention is not intended to be limited to the specific embodiments.

Preparation of phosphor particles 1: Large 14-hedron particles prepared by a method disclosed in Japanese Non-Examine Patent Publication 2003-246980
a) Phosphor particles 1 were prepared by
putting 1200 ml of BaBr₂ aqueous solution (1.5 moles/liter) in a 4000-ml reaction vessel (made of SUS316),
adding 37.5 ml of EuBr₃ aqueous solution (0.2 mole/liter), 0.9 g of KBr₃, 3.54 g of CaBr₂ 2H₂O, and 1762.5 ml of water to the above reaction vessel,
keeping the reaction mother liquid (BaBr₂ concentration of 1.00 mole/liter) in this reaction vessel at 60 °C,
stirring the reaction mother liquid by a 60 mm-diameter screw-type stirrer at 500 r.p.m.,
feeding 300 ml of NH₄F aqueous solution (5 moles/liter ) to the reaction mother liquid at a feed rate of 5.0 ml/minute by a roller pump while the liquid is being stirred at 60 °C,
keeping on stirring the liquid at 60 °C for 2 hours to age the precipitate,
separating the precipitate by filtration,
washing the precipitate with 2 liters of methanol,
transferring the clean precipitate to an evaporation pan,
and vacuum-drying the precipitate at 120 °C for 4 hours.

The product was approx. 350 g of europium-activated barium fluoride bromide phosphor precursor (BaFBr crystal).

The obtained phosphor precursor was further treated by
adding 0.2 mass percent of ultra-fine alumina powder to the product to prevent change in particle shapes by sintering (during calcinations) and change in the particle size distribution due to fusion-bonding of particles,
fully stirring the mixture by the mixer to attach ultra-fine alumina powder evenly to the surfaces of the crystals,
putting the mixture in a quartz boat,
placing the quartz boat in the tube furnace,
calcinating thereof at 850 °C for 2-hours in the hydrogen gas atmosphere,
and sieving the phosphor particles.

The obtained product was europium-activated barium fluoride bromide phosphor crystal (BaFBr crystal).

The most of the obtained crystals was 14-hedron crystals when observed through a scanning electron microscope.

The mean crystal size of the product was 8.0 µm when measured by a Horiba Optical Diffraction Type Particle Size Distribution Tester LA-500 (fabricated by Horiba, Ltd).

Preparation of phosphor particles 2: Small 14-hedron particles prepared by a method disclosed in Japanese Non-Examine Patent Publication 2003-246980
b) Phosphor particles 2 were prepared by
putting 2850 ml of BaI₂ aqueous solution (4.0 moles/liter) in a 4000-ml reaction vessel (made of SUS316),
adding 90 ml of EuI₃ aqueous solution (0.2 mole/liter) and 60 ml of water to the above reaction vessel,
keeping the reaction mother liquid (BaI₂ concentration of 3.80 moles/liter) in this reaction vessel at 50 °C,
stirring the reaction mother liquid by a 60 mm-diameter screw-type stirrer at 500 r.p.m.,
feeding 720 ml of HF aqueous solution (5 moles/liter ) to the reaction mother liquid at a feed rate of 12 ml/minute by a roller pump while the liquid is being stirred at 50 °C,
keeping on stirring the liquid at 50 °C for 2 hours to age the precipitate,
separating the precipitate by filtration,
washing the precipitate with 2 liters of isopropanol,
transferring the clean precipitate to an evaporation pan,
and vacuum-drying the precipitate at 120 °C for 4 hours.

The product was approx. 1,000 g of europium-activated barium fluoride iodide phosphor precursor (BaFI crystal).

The obtained phosphor precursor was further treated by
adding 0.2 mass percent of ultra-fine alumina powder to the product to prevent change in particle shapes by sintering (during calcinations) and change in the particle size distribution due to fusion-bonding of particles,
fully stirring the mixture by the mixer to attach ultra-fine alumina powder evenly to the surfaces of the crystals,
putting the mixture in a quartz boat,
placing the quartz boat in the tube furnace,
calcinating thereof at 850 °C for 2 hours in the hydrogen gas atmosphere,
and sieving the phosphor particles.

The obtained product was europium-activated barium fluoride iodide phosphor crystal (BaFI crystal).

The most of the obtained crystals was 14-hedron crystals when observed through a scanning electron microscope.

The mean crystal size of the product was 4.5 µm when measured by a Horiba Optical Diffraction Type Particle Size Distribution Tester.

Preparation of phosphor particles 3: Small hexahedron particles prepared by a method disclosed in Japanese Non-Examine Patent Publication 2003-268369

Photostimulable phosphor precursor of europium-activated barium fluoride iodide was prepared by
putting 2500 ml of BaI₂ aqueous solution (4 moles/liter) and 26.5 ml of EuI₃ aqueous solution (0.2 moles/liter) in a pressure-tight reaction vessel having two ventilation holes,
adding 992 g of potassium iodide to the above aqueous solution,
stirring the reaction mother liquid at 85 °C in the reaction vessel,
feeding dry air into the reaction vessel at a flow rate of 10 liters/minute,
adding 600 ml of aqueous solution of ammonium fluoride (10 moles/liter) into the reaction mother liquid in one hour by a roller pump while keeping the pressure in the reaction vessel at 36.3 kPa by a circulation aspirator to concentrate the solvent,
(wherein the produced precipitate after ventilation is 0.92 by mass of the produced precipitate before ventilation)
keeping on stirring the liquid at that temperature for 60 minutes,
filtering the liquid,
washing the precipitate on the filter with 2000 ml of ethanol,
drying thereof at 80 °C (wherein the product was approx. 1,000 g of europium-activated barium fluoride iodide phosphor precursor crystal (BaFI crystal)),
adding 0.2 mass percent of ultra-fine alumina powder to the product to prevent change in particle shapes by sintering (during calcinations) and change in the particle size distribution due to fusion-bonding of particles,
fully stirring the mixture by the mixer to attach ultra-fine alumina powder evenly to the surfaces of the crystals,
putting the mixture in a quartz boat,
placing the quartz boat in the tube furnace, and
calcinating thereof at 850 °C for 2 hours in the hydrogen gas atmosphere.

The obtained product was europium-activated barium fluoride iodide phosphor particles.

The most of the obtained crystals was hexahedron crystals when observed through a scanning electron microscope.

The mean crystal size of the product was 4.5 µm when measured by a Horiba Optical Diffraction Type Particle Size Distribution Tester.

Preparation of phosphor particles 4: Large hexahedron particles prepared by a method disclosed in Japanese Non-Examine Patent Publication 2003-268369

Photostimulable phosphor precursor of europium-activated barium fluoride iodide was prepared by
putting 2500 ml of BaI₂ aqueous solution (4 moles/liter) and 26.5 ml of EuI₃ aqueous solution (0.2 moles/liter) in a pressure-tight reaction vessel having two ventilation holes,
adding 992 g of potassium iodide to the above aqueous solution,
stirring the reaction mother liquid at 92 °C in the reaction vessel,
feeding dry air into the reaction vessel at a flow rate of 10 liters/minute,
adding 600 ml of aqueous solution of ammonium fluoride (10 moles/liter) into the reaction mother liquid in three hours by a roller pump while keeping the pressure in the reaction vessel at 42.3 kPa by a circulation aspirator to concentrate the solvent,
(wherein the produced precipitate after ventilation is 0.92 by mass of the produced precipitate before ventilation) keeping on stirring the liquid at that temperature for 60 minutes,
filtering the liquid,
washing the precipitate on the filter with 2000 ml of ethanol,
drying thereof at 80 °C (wherein the product was approx. 1,000 g of europium-activated barium fluoride iodide phosphor precursor crystal (BaFI crystal)),
adding 0.2 mass percent of ultra-fine alumina powder to the product to prevent change in particle shapes by sintering (during calcinations) and change in the particle size distribution due to fusion-bonding of particles,
fully stirring the mixture by the mixer to attach ultra-fine alumina powder evenly to the surfaces of the crystals,
putting the mixture in a quartz boat,
placing the quartz boat in the tube furnace, and
calcinating thereof at 850 °C for 2 hours in the hydrogen gas atmosphere.

The obtained product was europium-activated barium fluoride iodide phosphor particles.

The most of the obtained crystals was hexahedron crystals when observed through a scanning electron microscope.

The mean crystal size of the product was 8 µm when measured by a Horiba Optical Diffraction Type Particle Size Distribution Tester.

Preparation of phosphor particles 5: Large amorphous particles prepared by a method disclosed in Japanese Non-Examine Patent Publication 2004-138440

Photostimulable phosphor precursor of europium-activated barium fluoride iodide was prepared by
putting 2780 ml of BaI₂ aqueous solution (3.6 moles/liter) and 27 ml of EuI₃ aqueous solution (0.2 mole/liter) in a reaction vessel,
stirring the reaction mother liquid in the reaction vessel at 83 °C,
adding 322 ml of aqueous solution of ammonium fluoride (8 moles/liter) to the reaction mother liquid by a roller pump, and
keeping on stirring the liquid containing precipitate for 2 hours after addition of ammonium fluoride to age the precipitate.

The precipitate was further treated by
filtering the liquid,
washing the precipitate on the filter with ethanol,
vacuum-drying thereof (wherein the product was europium-activated barium fluoride iodide phosphor crystal),
adding 0.2 mass percent of ultra-fine alumina powder to the product to prevent change in particle shapes by sintering (during calcinations) and change in the particle size distribution due to fusion-bonding of particles,
fully stirring the mixture by the mixer to attach ultra-fine alumina powder evenly to the surfaces of the crystals,
putting the mixture in a quartz boat,
placing the quartz boat in the tube furnace, and
calcinating thereof at 850 °C for 2 hours in the hydrogen gas atmosphere.

The obtained product was europium-activated barium fluoride iodide phosphor particles.

By sieving thereof, the mean crystal size of the prepared product was 7 µm.

Preparation of phosphor 6 by a method disclosed in Japanese Non-Examine Patent Publication 2004-125398

Photostimulable phosphor precursor of europium-activated barium fluoride iodide was prepared by
putting 2780 ml of BaI₂ aqueous solution (3.6 moles/liter) and 27 ml of EuI₃ aqueous solution (0.15 mole/liter) in a reaction vessel,
stirring the reaction mother liquid in the reaction vessel at 83 °C,
adding 322 ml of aqueous solution of ammonium fluoride (8 moles/liter) to the reaction mother liquid by a roller pump,
keeping on stirring the liquid containing precipitate for 2 hours after addition of ammonium fluoride to age the precipitate.
filtering the liquid,
washing the precipitate on the filter with ethanol,
vacuum-drying thereof (wherein the product was europium-activated barium fluoride iodide phosphor crystal),
adding 0.2 mass percent of ultra-fine alumina powder to the product to prevent change in particle shapes by sintering (during calcinations) and change in the particle size distribution due to fusion-bonding of particles,
fully stirring the mixture by the mixer to attach ultra-fine alumina powder evenly to the surfaces of the crystals,
putting the mixture in a quartz boat,
placing the quartz boat in the tube furnace, and
calcinating thereof at 850 °C for 2 hours in the hydrogen gas atmosphere.

The obtained product was europium-activated barium fluoride iodide phosphor particles.

By sieving thereof, the mean crystal size of the prepared product was 4 µm.

Preparation of a phosphor layer coating liquid

A phosphor layer coating liquid having a viscosity of 25 to 30 mPa·s was prepared by
mixing a total of 480 g of ingredients listed in Table 1 including the prepared phosphor,
adding the mixture and 57.0 g of polyurethane resin whose Tg is 30 °C (NippoLan 2304 (solid content 35% ), fabricated by Nippon Polyurethane Industry Co., Ltd.) into a solvent mixture including 1 part of methylethylketone and 1 part of toluene, and
fully dispersing thereof by a propeller mixer.

Preparation of a coating liquid for undercoat-layers

The coating liquid having a viscosity of 500 mPa·s was prepared by
mixing 100 mass parts of polyester resin (VYLON 300 fabricated by Toyobo Co., Ltd.) and 5 mass parts of silane coupling agent (Υ-mercapto propyl trimethoxy silane),
adding, as a crosslinking agent, 10 mass parts of Collonate HX (fabricated by Nippon Polyurethane Industry Co., Ltd.) which is a multifunctional isocyanate compound into the above mixture,
fully mixing thereof,
adding this mixture into a solvent mixture containing one part of methylethylketone and one part of toluene, and
fully dispersing thereof by a propeller mixer.

Forming an undercoated layer
An undercoated layer specimen was prepared by
applying the above coating liquid for undercoated layers to the surface of a black carbon-blended polyethylene terephthalate base (backing) of 250 µm in thickness, controlling the thickness of the coating liquid layer to 15 µm by a knife coater, and drying thereof.

Heat-treating the undercoated specimen

The above undercoated specimen was heat-treated (or aged) at 60 °C for 100 hours.

Applying a phosphor layer

Photostimulable phosphor sheets were prepared by
applying the above prepared phosphor layer coating liquid to the heat-treat specimens and non-heat-treated specimens to form a 380 µm-thick dry layer on each of the specimen, and drying thereof at 100 °C for 30 minutes.

Some of the above coated and dried sheet specimens of photostimulable phosphor were compressed by a set of rolls of FIG. 2.

The compressing section comprises three rolls in series. Two nipping areas are formed by two heat rolls (9-1 and 9-3) and one compliant roll (9-2). The compressing section is controlled so that the compliant roll may touch the surface of the photostimulable phosphor layer. This compressing system comprises supply roll 6, takeup roll 10, dry zone 8, base sheet 7, and coater 4. The base sheet is delivered in the direction of D.

Heat rolls 9-1 and 9-3 are respectively 300 mm in diameter and 0.2S in surface. The compliant roll 9-2 is a 250 mm-diameter MirrorTex polyester roll (fabricated by Yamanouchi Rubber Co., Ltd.) which has a Shore hardness of D75°, a crown value of 0 µm, and a mean surface roughness Ra (on the center line, defined by JIS-B-0601) of 0.4 µm. The compressing was carried out at 70 °C (on the heat rolls) and 1 kN/cm (line pressure).

Measuring and calculating the phosphor filling factor

The phosphor filling factor of each phosphor sheet specimen was obtained by measuring the thickness of the phosphor layer on the specimen and calculating the phosphor filling factor (per 100 cm² of the phosphor sheet) by the above expression using the measured thickness and the specific gravity of phosphor (5.4 g/cm³).

Preparation of a moisture-proof protective film

A moisture-proof protective film below was prepared to protect the coated side of each of the phosphor sheet specimens 1 to 8.
Layer configuration (A)
NY15///VMPET12///VMPET12///PET12///CPP20
where
NY: Nylon
PET: Polyethylene terephthalate
CPP: Casted polypropylene
VMPET: Alumina-deposited PET (commercially available, fabricated by TORAY ADVANCED FILM Co., Ltd.)

A number that follows the name of each resin film represents the thickness (in µm) of the film.
"///" represents a dry lamination bonding layer of 3.0 µm in thickness. This bonding layer uses a 2-liquid reaction type urethane adhesive.

The back side of each phosphor sheet specimen is protected by a dry lamination film which laminates a 30 µm-thick CPP film, a 9 µm-thick aluminum film, and a 188 µm-thick polyethylene terephthalate (PET) film.

The laminated films are respectively bonded by a 1.5 µm-thick adhesive layer of a 2-liquid reaction type urethane adhesive.

### Preparation of phosphor plates

The above prepared phosphor sheet specimens are respectively cut into 20 cm-square sheets. Each square sheet is covered with one or two moisture-proof protective films. The edges of the protective films are heat-sealed by an impulse sealer in a pressure-reduced environment. Eight phosphor plate specimens 1 to 8 were prepared in this manner. The distance between the heat-sealed line and the outer edge of each phosphor sheet is 1 mm. The impulse sealer uses a 3 mm-wide heater.

### Evaluation of image sharpness

In FIG. 1, the evaluation of image sharpness was made by
placing a lead-made MTF charts (short for Modulation Transfer Function) 1 meter away from the X-ray source,
placing a phosphor plate in the X-ray detector 4 which is 1.7 meter away from the X-ray source to receive the MTF chart,
making an X-ray shot to the the MTF chart from the X-ray source at a tube voltage of 80 kVp,
exciting the exposed plate by He-Ne laser beams,
receiving photostimulable light from the phosphor layer by a photo acceptor (a photomultiplier of spectral sensitivity S-5),
converting the received light to an electric signal,
converting this analog electric signal to a digital signal,
recording the signal in magnetic tape,
analyzing the signal in the magnetic tape by a computer, and
examining the modulation transfer function (MTF) at 1 cycle/mm of the X-ray image which is recorded on the magnetic tape.

The result is defined as a degree of sharpness relative to the degree of sharpness (100) of phosphor plate 1.
Evaluation of graininess (or surface roughness)
Referring to FIG. 1,
the graininess evaluation was made by
placing a chest phantom 1 meter away from the X-ray source,
placing a phosphor plate in the X-ray detector 4 which is 1.7 meter away from the X-ray source to receive the phantom image,
making an X-ray shot to the chest phantom from the X-ray source at a tube voltage of 80 kVp,
exciting the exposed plate by He-Ne laser beams,
receiving photostimulable light from the phosphor layer by a photo acceptor in the same manner as [0107],
converting the received light to an electric signal,
reproducing an image from this signal by an image reproducing device,
reducing the image by 1/1.7,
printing out the reduced image, and
evaluating the iamge roughness by eyes.

The criterion below was used for evaluation of image roughness.
A: No image roughness recognized
B: A little but negligible image roughness recognized
C: Some but substantially permissible image roughness recognized
D: Unnegligible image roughness recognized on the entire surface
   Table 1 shows the result of evaluation.
   Evaluation of image defects
   Referring to FIG. 1,
   the image defect evaluation was made by
   placing a 5 mm-thick aluminum plate 1 meter away from the X-ray source,
   placing a phosphor plate in the X-ray detector 4 which is 1.7 meter away from the X-ray source to receive the aluminum plate image,
   making an X-ray shot to the aluminum plate from the X-ray source at a tube voltage of 80 kVp,
   exciting the exposed plate by He-Ne laser beams,
   receiving photostimulable light from the phosphor layer by a photo acceptor in the same manner as [0107],
   converting the received light to an electric signal,
   reproducing an image from this signal by an image reproducing device,
   reducing the image by 1/1.7,
   printing out the reduced image, and
   evaluating the image defects by eyes.

The criterion below was used for evaluation of image defects.
A: No image defects recognized
B: A little but negligible imagedefects recognized
C: Some but substantially permissible image defects recognized
D: Unnegligible image defects recognized on the entire surface

Table 1 shows the result of evaluation.

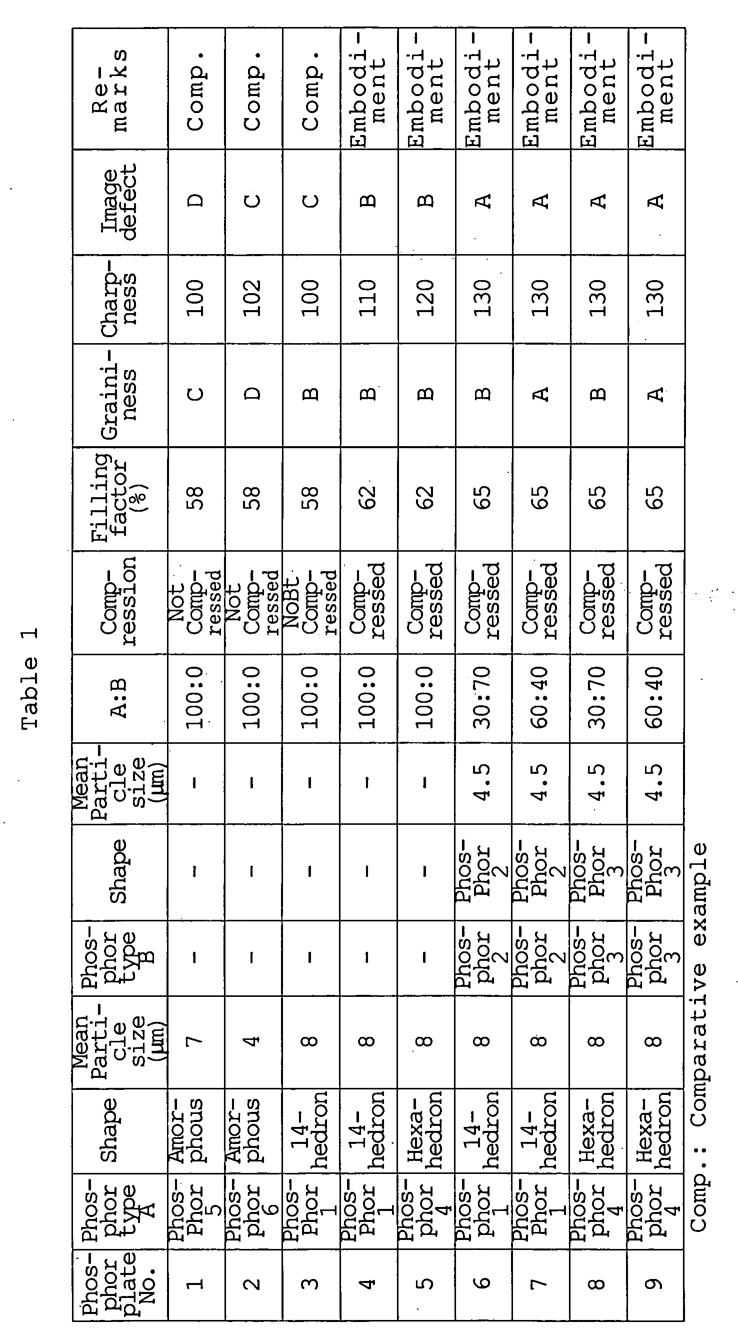

As evidenced by the above, the phosphor plates of this invention are superior in various radiographic characteristics to the comparative phosphor plates.

## Claims

1. A radiographic imaging device comprising a short-focus radiation source which radiates radiant rays to an examined object, a member to hold the examined object, and a radiographic image detector which detects radiant rays passing through the examined object and reads radiographic image information therefrom in order to perform phase contrast photography on the object which is held by the holding member, wherein
said detector is equipped with a phosphor plate which contains photostimulable phosphor particles having polyhedron crystal structures and the photostimulable phosphor occupies 60 to 80% of the photostimulable phosphor layer on the phosphor plate.

2. The radiographic imaging device of claim 1, wherein said photostimulable phosphor particles contain at least two different mean-particle-sizes of photostimulable phosphor particles.

3. The radiographic imaging device of claim 2, wherein the greater one of said 2 or more mean-particle-sizes of photostimulable phosphor particles is 6.0 to 10.0 µm and the smaller one is 3.0 to 5.0 µm.

4. The radiographic imaging device of claim 2, wherein the mixture ratio (by weight) of greater and smaller mean-particle-sizes of photostimulable phosphor particles is 95 : 5 to 50 : 50.

5. The radiographic imaging device of claim 1, wherein said photostimulable phosphor is represented by empirical formula (1) below.
Empirical formula (1)
Ba1-x M2x F Bry I1-y: aM1, bLn, cO
where
M1: at least one alkali metal atom selected from a group of Li, Na, K, Rb and Cs
M2: at least one alkali earth metal atom selected from a group of Be, Mg, Sr and Ca
Ln: at least one rare earth element selected from a group of Ce, Pr, Sm, Eu, Gd, Tb, Tm, Dy, Ho, Nd, Er and Yb
x, y, a, b and c: 0 ≤ x ≤ 0.3, 0 ≤ y ≤ 1, 0 ≤ a ≤ 0.05, 0 < b ≤ 0.2, 0 < c ≤ 0.1

6. A radiographic imaging method of shooting by said radiographic imaging device of claim 1 and reducing the recorded magnified image to the actual object size before outputting it.
